(19) Europäisches Patentamt
European Patent Office
Office européen des brevets

(11) Veröffentlichungsnummer: **0 215 424 A2**

(12) # EUROPÄISCHE PATENTANMELDUNG

(21) Anmeldenummer: **86112460.0**

(22) Anmeldetag: **09.09.86**

(51) Int. Cl.⁴: **C 07 D 209/46**, C 07 D 209/48, C 07 D 209/50, C 07 D 413/04, C 07 F 9/65, A 01 N 43/38, A 01 N 57/08, A 01 N 43/84

(30) Priorität: **19.09.85 DE 3533442**

(43) Veröffentlichungstag der Anmeldung: **25.03.87 Patentblatt 87/13**

(84) Benannte Vertragsstaaten: **AT BE CH DE FR GB IT LI NL SE**

(71) Anmelder: **HOECHST AKTIENGESELLSCHAFT, Postfach 80 03 20, D-6230 Frankfurt am Main 80 (DE)**

(72) Erfinder: **Liebl, Rainer, Dr., An der Weinleite 5b, D-8901 Todtenweis (DE)**
Erfinder: **Handte, Reinhard, Dr., Theilweg 23, D-8901 Gablingen (DE)**
Erfinder: **Mildenberger, Hilmar, Dr., Fasanenstrasse 24, D-6233 Kelkheim (Taunus) (DE)**
Erfinder: **Bauer, Klaus, Dr., Kolpingstrasse 7, D-6054 Rodgau (DE)**
Erfinder: **Bieringer, Hermann, Dr., Eichenweg 26, D-6239 Eppstein/Taunus (DE)**

(54) **Neue 2-Aryl-4,5,6,7-tetrahydroisoindolinone, Verfahren zu ihrer Herstellung sowie ihre Verwendung im Pflanzenschutz.**

(57) Die Verbindungen der Formel

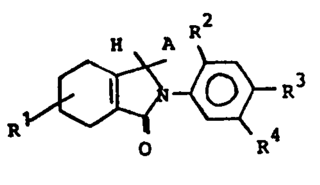

worin
$R^1$ = H oder Alkyl, $R^2$ = Wasserstoff oder Halogen, $R^3$ = Halogen, $R^4$ = Hydroxy, Alkoxy, Alkoxyalkoxy, Cycloalkoxy, Alkenyloxy, Alkinyloxy, Alkylthio, Alkylsulfinyl, Alkylsulfonyl, $NO_2$, CN, Halogen oder Cyanoalkyl, Phenoxy, Phenoxyalkyl, Phenylalkoxy, Phenylthio, Phenylsulfinyl, Phenylsulfonyl, wobei in den 6 letztgenannten Resten der Phenylring substituiert sein kann, eine Gruppe der Formeln

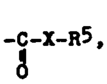, 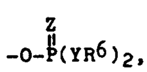, 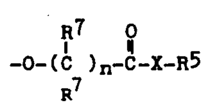 oder

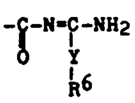,

X = O, S oder $NR^8$, Y, Z = O oder S, $R^5$ = H, (subst.) Alkyl, Cycloalkyl, Alkenyl, Cyclohexenyl, Alkinyl, (subst.) Phenyl, einen Rest der Formel $-N=CR^9R^{10}$ oder ein in der Landwirtschaft einsetzbares Kation, $R^6$ = Alkyl, Benzyl, $R^7$ = Wasserstoff, Alkyl oder $-CCl_3$, $R^8$ = Wasserstoff, $(C_1-C_4)$-Alkyl, das zusammen mit $R^5$ und dem diese Reste verbindenden Stickstoffatom einen Heterocyclus bilden kann, $R^9$, $R^{10}$ = Alkyl, Cycloalkyl, Alkoxycarbonyl oder gemeinsam eine Alkylenkette; A=OH SH, Halogen, CN oder einen Rest der Formeln $-OR^{11}$, $-S(O)_p-R^{11}$, $-NR^{12}R^{13}$ oder $-NH-NR^{14}R^{15}$ und n = die Zahl 1, 2 oder 3 und p = die Zahl 0, 1 oder 2 bedeuten, besitzen vorteilhafte herbizide Eigenschaften gegenüber mono- und dikotylen Schadpflanzen.

EP 0 215 424 A2

Neue 2-Aryl-4,5,6,7-tetrahydroisoindolinone, Verfahren zu ihrer Herstellung sowie ihre Verwendung im Pflanzenschutz

Es ist bekannt, daß 4,5,6,7-Tetrahydroisoindolinone herbizide Eigenschaften aufweisen, s. EP-A-40849, EP-A-41256, DE-OS 2831770.

Die herbizide Aktivität und die Selektivität dieser Verbindungen ist jedoch nicht immer voll befriedigend.

Es wurden nun neue 4,5,6,7-Tetrahydroisoindolinone gefunden, die überraschenderweise über eine wesentlich stärkere selektive Herbizid-Wirkung verfügen.

Gegenstand der Erfindung sind daher die Verbindungen der Formel I

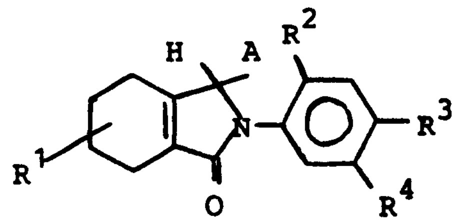

(I),

worin

$R^1$ = $(C_1-C_4)$-Alkyl oder Wasserstoff

$R^2$ = Wasserstoff oder Halogen

$R^3$ = Halogen

$R^4$ = Hydroxy, $(C_1-C_4)$-Alkoxy, $(C_1-C_4)$Alkoxy-$(C_1-C_4)$-alkoxy, $(C_3-C_6)$-Cycloalkoxy, $(C_3-C6)$-Alkenyloxy, $(C_3-C_6)$-Alkinyloxy, $(C_1-C_4)$-Alkylthio, $(C_1-C_4)$-Alkylsulfinyl, $(C_1-C_4)$-Alkylsulfonyl, $NO_2$, CN, Halogen-$(C_1-C_4)$-alkyl mit einem oder mehreren Halogenatomen, Mono-Cyano-$(C_1-C_4)$-alkyl, Phenoxy, Phenoxy-$(C_1-C_4)$alkyl, Phenyl-$(C_1-C_4)$-alkoxy Phenylthio, Phenylsulfinyl, Phenylsulfonyl, wobei in den 6 letztgenannten Resten der Phenylring bis zu dreifach durch Halogen, $(C_1-C_4)$-Alkyl, $(C_1-C_4)$-Alkoxy oder $(C_1-C_4)$-Alkoxycarbonyl substituiert

sein kann,

eine Gruppe der Formeln

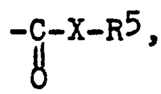, 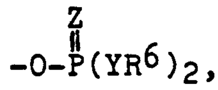, 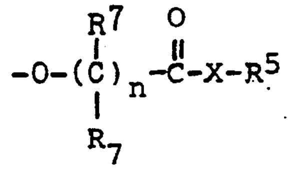 oder

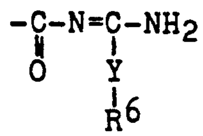 ,

X = O, S oder $NR^8$,

Y,Z = unabhängig voneinander O oder S

$R^5$ = Wasserstoff, $(C_1-C_{12})$-Alkyl, das bis zu 6-fach durch Halogen und/oder bis zu dreifach durch $(C_1-C_4)$-Alkoxy, $(C_1-C_4)$-Alkoxy-$(C_1-C_4)$-alkoxy, $(C_1-C_4)$-Alkoxycarbonyl, $(C_1-C_4)$-Alkylthio, $(C_1-C_4)$-Alkylsulfonyl, $(C_1-C_4)$-Alkylsulfinyl, $(C_1-C_4)$-Alkylamino, Di- $(C_1-C_4)$-alkylamino, Furyl, Tetrahydrofuryl, Benzofuryl, Phenyl, Phenoxy, Benzyloxy, wobei die drei letztgenannten Reste im Phenylteil bis zu zweifach durch Halogen, $(C_1-C_4)$-Alkyl oder $(C_1-C_4)$-Alkoxy substituiert sein können, substituiert sein kann, $(C_3-C_6)$-Cycloalkyl, $(C_3-C_6)$-Alkenyl, Cyclohexenyl, $(C_3-C_6)$-Alkinyl, Phenyl, das bis zu dreifach durch Halogen, $(C_1-C_4)$-Alkyl, $(C_1-C_4)$-Alkoxy oder $(C_1-C_4)$-Alkoxycarbonyl substituiert sein kann,

ein in der Landwirtschaft einsetzbares Kation oder einen Rest der Formel $-N=CR^9R^{10}$, wobei für den letztgenannten Rest $R^4 = -CO-OR^5$ bedeuten muß,

$R^6$ = unabhängig voneinander $(C_1-C_4)$-Alkyl oder Benzyl

$R^7$ = unabhängig voneinander Wasserstoff, $(C_1-C_4)$-Alkyl oder $-CCl_3$,

$R^8$ = Wasserstoff, $(C_1-C_4)$-Alkyl, das zusammen mit $R^5$ und dem diese Reste verbindenen Stickstoffatom einen fünf- bis siebengliedrigen Heterocyclus bilden kann, der als Ringglieder 1 oder 2 Reste der Gruppe -O-, -S- oder $-NR^6$ enthält und der bis zu dreifach durch $(C_1-C_4)$-Alkyl substituiert sein kann,

$R^9$,$R^{10}$= unabhängig voneinander $(C_1-C_4)$-Alkyl, $(C_3-C_6)$-Cycloalkyl, $(C_1-C_4)$Alkoxycarbonyl oder gemeinsam eine $(C_2-C_7)$-Alkylenkette,

A = OH, SH, Halogen, CN, einen Rest der Formeln $-OR^{11}$, $-S(O)_pR^{11}$, $-NR^{12}R^{13}$ oder $-NH-NR^{14}R^{15}$,

$R^{11}$ = $(C_1-C_{12})$-Alkyl, $(C_1-C_4)$-Alkoxy-$(C_1-C_4)$-alkyl, Phenoxy$(C_1-C_4)$alkyl, Benzyloxy$(C_1-C_4)$alkyl, wobei die beiden letztgenannten Reste im Phenylring bis zu dreifach durch Halogen, $(C_1-C_4)$-Alkyl oder $(C_1-C_4)$-Alkoxy substituiert sein können, $(C_1-C_4)$-Alkoxycarbonyl-$(C_1-C_4)$-alkyl, Phenyl oder Benzyl, die beide bis zu dreifach im Phenylring durch Halogen, $(C_1-C_4)$-Alkyl, $(C_1-C_4)$-Alkoxy, $(C_1-C_4)$-Alkoxycarbonyl, $(C_1-C_4)$-Alkoxycarbonyl-$(C_1-C_4)$-alkoxy, $CF_3$ oder $NO_2$ substituiert sein können,
eine Gruppe der Formeln
$-C(=O)-R^{16}$, $-C(=O)-OR^{16}$ oder $-C(=O)-NH-R^{16}$,

$R^{12}$,$R^{13}$=unabhängig voneinander Wasserstoff, $(C_1-C_4)$-Alkyl, oder zusammen mit dem sie verbindenden Stickstoffatom einen 3- bis 7-gliedrigen Ring bilden, wobei eine Methylengruppe durch O, S oder $NR^7$ ersetzt sein kann und der bis zu dreifach durch $(C_1-C_4)$-Alkyl substituiert sein kann,
Phenyl oder Benzyl, die beide im Phenylring bis zu dreifach durch Halogen, $(C_1-C_4)$-Alkyl, $(C_1-C_4)$-Alkoxy, $(C_1-C_4)$-Alkoxycarbonyl, $CF_3$ oder $NO_2$ substituiert sein können,

$R^{14}$,$R^{15}$=unabhängig voneinander Wasserstoff, $(C_1-C_4)$-Alkyl oder Phenyl, das bis zu dreifach durch Halogen oder $(C_1-C_4)$-Alkyl substituiert sein kann,

$R^{16}$ = $(C_1-C_4)$-Alkyl, das bis zu dreifach durch Halogen, $(C_1-C_4)$-Alkoxy, $(C_1-C_4)$-Alkylthio, CN, Phenyl oder

Phenyl, das bis zu zweifach durch Halogen, $(C_1-C_4)$-Alkyl, $(C_1-C_4)$-Alkoxy, $CF_3$, $NO_2$ substituiert ist, substituiert sein kann, oder Phenyl, das ein- bis dreifach durch Halogen, $(C_1-C_4)$-Alkyl, $(C_1-C_4)$-Alkoxy, $(C_1-C_4)$-Alkoxycarbonyl, $CF_3$ oder $NO_2$ substituiert sein kann,

n = die Zahl 1, 2 oder 3 und

p = die Zahl 0, 1 oder 2

bedeuten.

Halogen bedeutet insbesondere F, Cl oder Br. Als für die Landwirtschaft einsetzbare Kationen kommen beispielsweise in Betracht Alkali-, Erdalkalikationen wie Na, K, Ca, Mg-Ionen, oder gegebenenfalls substituierte Ammoniumkationen.

Als Heterocyclen kommen im Falle $R^8-\overset{|}{N}-R^5$ oder im Falle $-NR^{12}R^{13}$ bevorzugt infrage: Morpholin, Piperazin, Piperidin, Pyrrolidin.

Die Verbindungen der Formel I können auch als reine Stereoisomeren (z.B. im Falle $R^4 = -O[C(R^7)_2]_n-CO-X-R^5$) oder als deren Gemische vorliegen. Alle diese Isomeren werden von vorliegender Erfindung umfaßt.

Von den Verbindungen der Formel I sind bevorzugt solche, bei denen

$R^2$ = H, F oder Cl,

$R^3$ = Cl oder Br,

$R^4$ = $-CO-X-R^5$, $-O-\left(\begin{matrix} R^7 \\ | \\ -C- \\ | \\ R^7 \end{matrix}\right)_n-CO-X-R^5$, $(C_1-C_4)$Alkoxy-$(C_1-C_4)$-alkoxy und im Falle $R_2$= F auch $(C_1-C_4)$Alkoxy,

$R^5$ = $(C_1-C_6)$Alkyl, das durch $(C_1-C_4)$Alkoxy oder $(C_1-C_4)$-Alkoxycarbonyl substituiert sein kann oder $(C_3-C_4)$Alkinyl,

$R^7$ = beide unabhängig voneinander $(C_1-C_4)$Alkyl oder ein

Rest $R^7$= H und der andere Rest $R^7$= $(C_1-C_4)$Alkyl oder $-CCl_3$,

$R^8$ = $(C_1-C_4)$Alkyl,

A = OH, Cl oder $SR^{11}$,

$R^{11}$= $(C_1-C_6)$Alkyl, $(C_1-C_4)$Alkoxycarbonyl$(C_1-C_4)$alkyl, Phenyl oder Benzyl, die beide bis zu 3-fach wie oben angegeben substituiert sein können,

X = O, S und

n = 1

bedeuten.

Gegenstand der vorliegenden Erfindung sind auch Verfahren zur Herstellung der Verbindungen der Formel I, dadurch gekennzeichnet, daß man

a) eine Verbindung der Formel II

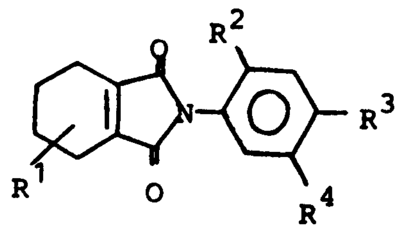

(II)

reduziert und

b) die unter a) erhaltenen Verbindungen der Formel I mit A = OH gegebenenfalls durch Umsetzung $b_1$) mit einer Verbindung der Formel $Cl-CO-R^{16}$, $Cl-CO-OR^{16}$ oder $R^{16}-NCO$ in Gegenwart einer Base oder $b_2$) mit einer Verbindung der Formel $R^{11}$-Hal (Hal = Cl, Br, J) in Gegenwart einer Base, oder $b_3$) mit einem Isocyanat $R^{16}-N=C=O$ gegebenenfalls in Gegenwart einer Base, oder $b_4$) mit einem anorganischen Säurechlorid wie $SOCl_2$, $POCl_3$, $PCl_3$ in andere Verbindungen der Formel I überführt und die unter $b_4$) erhaltenen Verbindungen der Formel I gegebenenfalls mit einem Alkalimetallfluorid, -bromid oder -jodid oder mit einem Alkalicyanid oder mit einer Verbindung der Formel $HOR^{11}$, $HSR^{11}$, $HNR^{12}R^{13}$, oder $H_2N-NR^{14}R^{15}$ umsetzt oder die unter $b_1$) erhaltenen Verbindungen im Falle A = $S-R^{11}$ gegebenenfalls oxidiert.

Im Falle der Reduktion (Verfahrensvariante a) wird als Reduktionsmittel vorzugsweise ein Metallhydrid wie z.B. $NaBH_4$ in einem organischen Lösungsmittel eingesetzt, wie z.B. Methanol, Ethanol oder auch Ether, Tetrahydrofuran und Dimethoxyethan bei Temperaturen zwischen 0 und 80°C, vorzugsweise bei 20 - 40°C, s.a. Rocz. Chem., 49, 1671 (1975).

Die Verbindungen der allgemeinen Formel II sind zum Teil bekannt, s. Chem Ber. (1903), S. 996-1007), oder lassen sich einfach nach üblichen Methoden aus den entsprechenden Anilinen mit 3,4,5,6-Tetrahydrophthalsäureanhydrid herstellen.

Bei der Verfahrensvariante $b_1$) und $b_2$) wird als Base eine anorganische Base wie beispielsweise $K_2CO_3$ oder eine organische Base, wie beispielsweise $NEt_3$ oder Pyridin eingesetzt. Bei der Verfahrensvariante $b_3$) kann als Base ein organisches Amin wie Triethylamin oder 1,4-Diazabicyclo-(2,2,2)octan eingesetzt werden. Die unter $b_4$) genannte Umsetzung mit einem Alkalimetallfluorid-, -bromid-, -jodid oder -cyanid erfolgt vorzugsweise mittels Phasentransferkatalyse; zur Phasentransferkatalyse s. H. Böhme, U.Sitorus, Chemiker Ztg. 95 37 (1972). Als Alkalimetallverbindung wird bevorzugt die Na oder K-Verbindung eingesetzt. Die Oxidation der Verbindungen der Formel I mit A = $SR^{11}$ erfolgt in bekannter Weise z.B. mit Wasserstoffperoxid oder mit m-Chlorperbenzoesäure, s. Houben-Weyl, Methoden der organischen Chemie Bd IX S. 211, 227.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Beispielsweise können folgende Schadpflanzen bekämpft werden:
Schadgräser wie
Avena fatua, Alopecurus sp., Lolium sp., Setaria sp., Digitaria sp., Sorghum halepense, Echinochloa sp., Agropyron sp., Cynodon sp., Phalaris sp.,

Dikotyle Pflanzen wie
Lamium sp., Veronica sp., Galium sp., Stellaria sp., Matricaria sp., Papaver sp., Centauria sp., Amaranthus sp., Galinsoga sp., Mercurialis sp., Sida sp., Abutilon sp., Ambrosia sp., Xanthium sp., Cirsium sp., Artemisia sp., Rumex sp., Convolvulus sp., Ipomea sp., Sinapis sp.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutyl-

...

naphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen,z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemit-

...

telgranulaten üblichen Weise, gewünschtenfalls in Mischung mit Düngemitteln, granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 2 kg/ha.

Auch Mischungen oder Mischformulierungen mit anderen Wirkstoffen, wie z. B. Insektiziden, Akariziden, Herbiziden,

...

Düngemitteln, Wachstumsregulatoren oder Fungiziden sind gegebenenfalls möglich.

Die Erfindung wird durch nachstehende Beispiele erläutert:

A. Formulierungsbeispiele

Ein Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## B. Chemische Beispiele

### Beispiel 1

**2-(4-Chlor-2-fluor-5-methoxy-phenyl)-3-hydroxy-4,5,6,7-tetrahydroisoindolinon**

46,5 g (0,15 mol) N-(4-Chlor-2-fluor-5-methoxy-phenyl)-3,4,5,6-tetrahydrophthalimid werden in 200 ml Methanol gelöst. Man gibt 7,6 g (0,20 mol) $NaBH_4$ so zu, daß die Temperatur auf 30 - 35 °C ansteigt. Nach beendeter Zugabe wird noch 30 min bei RT gerührt und anschließend auf 800 ml Eiswasser gegossen. Der ausgefallene Niederschlag wird abgesaugt und getrocknet. Der getrocknete Niederschlag wird mit 100 ml Ether 10 min aufgekocht, im Eisbad gekühlt und erneut abgesaugt. Man erhält 39,3 g (84 % d. Th.) 2-(4-Chlor-2-fluor-5-methoxy-phenyl)-3-hydroxy-4,5,6,7-tetrahydroisoindolinon in Form eines hellgrauen Pulvers mit Schmp. 194 - 197 °C.

### Beispiel 2

**3-Chlor-2-(4-chlor-2-fluor-5-methoxy-phenyl)-4,5,6,7-tetrahydroisoindolinon**

31,2 g (0,1 mol) 2-(4-Chlor-2-fluor-5-methoxy-phenyl)-3-hydroxy-4,5,6,7-tetrahydroisoindolinon (Beispiel 1) werden in 150 ml Methylenchlorid gelöst. Bei RT[1] tropft man 13,1 g (0,11 mol) Thionylchlorid zu. Man rührt 30 min bei RT und 1 h bei 40 °C. Die klare gelbe Lösung wird mit 100 ml $H_2O$ gewaschen, über $Na_2SO_4$ getrocknet und zur Trockene eingeengt. Man erhält 32,4 g (98 % d. Th.) 3-Chlor-2-(4-chlor-2-fluor-5-methoxy-phenyl)-4,5,6,7-tetrahydroisoindolinon in Form eines blaßgrünen Pulvers mit Schmp. 102-105 °C.

1) RT = Raumtemperatur

...

Beispiel 3

2-(4-Chlor-2-fluor-5-methoxy-phenyl)-3-phenylmercapto-4,5,6,7-tetrahydroisoindolinon

33,0 g (0,1 mol) 3-Chlor-2-(4-chlor-2-fluor-5-methoxy-phenyl)-4,5,6,7-tetrahydroisoindolinon (Bsp. 2 ) und 10,0 g (0,1 mol) $NEt_3$ werden in 200 ml Toluol gelöst. Man tropft 11,0 g (0,1 mol) Thiophenol zu und rührt 4 h bei 60 - 70 °C. Das ausgefallene Ammoniumchlorid wird abgesaugt, die Mutterlauge 2mal mit 100 ml $H_2O$ gewaschen, über $Na_2SO_4$ getrocknet und eingedampft . Man erhält 38,0 g (97 % d. Th.) 2-(4-Chlor-2-fluor-5-methoxy-phenyl)-3-phenylmercapto-4,5,6,7-tetrahydroisoindolinon in Form eines hellbraunen Öles.

Beispiel 4

2-(4-Chlor-2-fluor-5-methoxy-phenyl)-3-[4-(1-ethoxycarbonyl-ethoxy)-phenoxy]-4,5,6,7-tetrahydroisoindolinon

15,8 g (0,05 mol) 3-Chlor-2-(4-chlor-2-fluor-5-methoxy-phenyl)-4,5,6,7-tetrahydroisoindolinon (Bsp. 2 ) in 50 ml Acetonitril tropft man bei 20 °C zu einer Lösung von 10,5 g (0,05 mol) 2-(4-Hydroxy-phenoxy)-propionsäureethylester in 100 ml Acetonitril. Bei RT gibt man portionsweise 1,5 g 80%iges Natriumhydrid zu. Man rührt 4 h bei 50 °C, gießt auf 300 ml Eiswasser und extrahiert mit 150 ml Methylenchlorid. Die Methylenchloridphase wird mit 150 ml $H_2O$ gewaschen, über $Na_2SO_4$ getrocknet und eindampft. Man erhält 23,2 g (95 % d. Th.) 2-(4-Chlor-2-fluor-5-methoxy-phenyl)- 3-[4-(1-ethoxycarbonyl-ethoxy)-phenoxy]-4,5,6,7-tetrahydroisoindolinon in Form eines hellbraunen Harzes.

...

Beispiel 5

3-Acetyloxy-2-(4-chlor-2-fluor-5-methoxy-phenyl)-4,5,6,7-tetrahydroisoindolinon

31,2 g (0,1 mol) 2-(4-Chlor-2-fluor-5-methoxy-phenyl)-3-hydroxy-4,5,6,7-tetrahydroisoindolinon (Bsp. 1) werden in 200 ml Dimethoxyethan gelöst. Bei 10 °C gibt man portionsweise 3,0 g Natriumhydrid (80%ig) zu und rührt 30 min bei 10 °C. Bei 10 °C tropft man 7,9 g (0,1 mol) Acetylchlorid zu und rührt anschließend 2 h bei 40 °C. Man gießt auf 400 ml Wasser und extrahiert mit 200 ml Ether. Nach Trocknen der organ. Phase über $Na_2SO_4$ und abdestillieren des Lösungsmittels erhält man 32,1 g (95 % d. Th.) 3-Acetyloxy-2-(4-chlor-2-fluor-5-methoxy-phenyl)-4,5,6,7-tetrahydroisoindolinon in Form eines hellbraunen Öls.

Beispiel 6

2-(4-Chlor-2-fluor-5-methoxy-phenyl)-3-methoxycarbonyloxy-4,5,6,7-tetrahydroisoindolinon

31,2 g (0,1 mol) 2-(4-Chlor-2-fluor-5-methoxy-phenyl)-3-hydroxy-4,5,6,7-tetrahydroisoindolinon (Bsp. 1) und 8,7 g Chlorameisensäuremethylester werden in 150 ml Dimethoxyethan gelöst. Bei 10 °C gibt man portionsweise 3,0 g Natriumhydrid (80%ig) zu und rührt anschließend noch 2 h bei 30 °C. Man gießt auf 400 ml Eiswasser und extrahiert mit Ether. Man erhält 32,4 g (92 % d. Th.) 2-(4-Chlor-2-fluor-5-methoxy-phenyl)-3-methoxycarbonyloxy-4,5,6,7-tetrahydroisoindolinon in Form eines hellbraunen Öls.

...

Beispiel 7

2-(4-Chlor-2-fluor-5-methoxy-phenyl)-3-methylcarbamoyloxy-4,5,6,7-tetrahydroisoindolinon

Man löst 31,2 g (0,1 mol) 2-(4-Chlor-2-fluor-5-methoxy-phenyl)-3-hydroxy-4,5,6,7-tetrahydroisoindolinon (Bsp. 1) und 6,3 g (0,11 mol) Methylisocyanat in 200 ml Acetonitril und gibt 100 mg DABCO (1,4-Diazabicyclo[2.2.2]octan) zu. Nach 12 h Rühren bei RT wird das Lösungsmittel abdestilliert. Der verbleibende Rückstand wird in Ether aufgeschlämmt und abgesaugt. Man erhält 24,2 g (66 % d. Th.) 2-(4-Chlor-2-fluor-5-methoxy-phenyl)-3-methoxycarbamoyloxy-4,5,6,7-tetrahydroisoindolinon in Form farbloser Kristalle mit Schmp. 148 - 151 °C (Methanol).

Beispiel 8

2-(4-Chlor-2-fluor-5-methoxy-phenyl)-3-diethylamino-4,5,6,7-tetrahydroisoindolinon

Zu 33,0 g (0,1 mol) 3-Chlor-2-(4-chlor-2-fluor-5-methoxy-phenyl)-4,5,6,7-tetrahydroisoindolinon (Bsp. 2 ) in 200 ml Methylenchlorid tropft man bei 20 °C 14,6 g (0,2 mol) Diethylamin. Man rührt 4 h bei 20 - 25 °C, gibt 200 ml $H_2O$ dazu und trennt die organische Phase ab. Nach Trocknen über $Na_2SO_4$ wird das Lösungsmittel abdestilliert. Man erhält 32,5 g (92 % d. Th.) 2-(4-Chlor-2-fluor-5-methoxy-phenyl)-3-diethylamino-4,5,6,7-tetrahydroisoindolinon in Form eines gelben Öles.

...

Beispiel 9

2-(4-Chlor-2-fluor-5-methoxy-phenyl)-3-fluor-4,5,6,7-tetrahydroisoindolinon

33,0 g (0,1 mol) 3-Chlor-2-(4-chlor-2-fluor-5-methoxy-phenyl)-4,5,6,7-tetrahydroisoindolinon (Bsp. 2 ) wird in 250 ml trockenem Acetonitril gelöst. Man gibt 14,5 g (0,25 mol) KF und 100 mg Kronenether (18-crown-6) zu und rührt 5 h bei 40 °C. Der Ansatz wird auf 600 ml Wasser gegossen und mit Methylenchlorid extrahiert. Nach Abdestillieren des Acetonitrils erhält man 28,4 g (91 % d. Th.) 2-(4-Chlor-2-fluor-5-methoxy-phenyl)-3-fluor-4,5,6,7-tetrahydroisoindolinon in Form blaßgelber Kristalle mit Schmp. 88-90 °C.

In analoger Weise erhält man die Verbindungen der folgenden Tabelle 1.

...

Tabelle 1

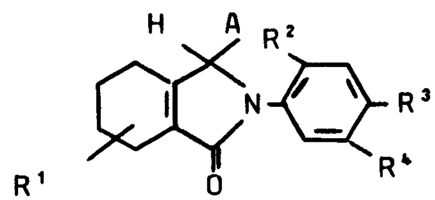

| Nr. | $R^1$ | $R^2$ | $R^3$ | $R^4$ | A | Fp [°C] | Herstellung analog Bsp. |
|---|---|---|---|---|---|---|---|
| 10 | H | F | Cl | $-O-\overset{OC_2H_5}{\underset{OC_2H_5}{P}}=S$ | -OH | gelbes Oel | 1 |
| 11 | H | F | Cl | $-O-C_3H_7(i)$ | -OH | 78-91 | 1 |
| 12 | H | F | F | $-CO_2C_2H_5$ | -OH | 181-183 | 1 |
| 13 | H | Cl | Cl | $-CO_2CH_3$ | -OH | 148-151 | 1 |
| 14 | H | Cl | Cl | $-CO_2C_2H_6$ | -OH | | 1 |
| 15 | H | Cl | Cl | $-CO_2C_4H_9$ | -OH | gelbes Oel | 1 |
| 16 | H | Cl | Cl | $-\underset{\overset{\|}{O}}{C}-O-\overset{CH_3}{CH}-CO_2C_2H_5$ | -OH | " " | 1 |
| 17 | H | F | Cl | -OH | -OH | 188-192 | 1 |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 18 | H | H | Cl | $-CO_2C_2H_5$ | -OH | 149-154 | 1 |
| 19 | H | Cl | Cl | $-OCH_3$ | -OH | 172-174 | 1 |
| 20 | H | Cl | Cl | $-C(=O)-O-CH_2-CO_2CH_3$ | -OH | | 1 |
| 21 | H | Cl | Cl | 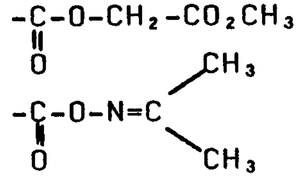 | -OH | | 1 |
| 22 | H | Cl | Cl | $-C(=O)-N=C(OCH_3)-NH_2$ | -OH | | 1 |
| 23 | H | F | Cl | $-O-P(=O)(OC_2H_5)_2$ | -Cl | hellbraunes Oel | 2 |
| 24 | H | F | Cl | $-O-C_3H_7(i)$ | -Cl | dto. | 2 |
| 25 | H | F | F | $-CO_2C_2H_5$ | -Cl | dto. | 2 |
| 26 | H | Cl | Cl | $-CO_2CH_3$ | -Cl | dto. | 2 |
| 27 | H | Cl | Cl | $-CO_2C_2H_5$ | -Cl | dto. | 2 |
| 28 | H | Cl | Cl | $-C(=O)-O-CH(CH_3)-CO_2C_2H_5$ | -Cl | gelbes Oel | 2 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 29 | H | Cl | Cl | 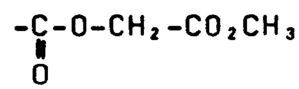 | -Cl | gelbes Oel | 2 |
| 30 | H | Cl | Cl | $-OCH_3$ | -Cl | braunes Harz | 2 |
| 31 | H | H | Cl | $-CO_2C_2H_5$ | -Cl | dto. | 2 |
| 32 | H | Cl | Cl | 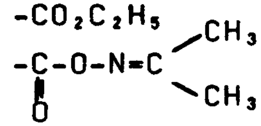 | -Cl | gelbes Oel | 2 |
| 33 | H | F | Cl | $-OC_3H_7(i)$ | $-S-C_6H_5$ | hellbraunes Oel | 3 |
| 34 | H | F | Cl | $-OCH_3$ | $-S-C_2H_5$ | " | 3 |
| 35 | H | F | Cl | $-OC_3H_7(i)$ | $-S-C_2H_5$ | gelbes Oel | 3 |
| 36 | H | F | Cl | $-OCH_3$ | $-S-(4-Cl-C_6H_4)$ | " | 3 |
| 37 | H | F | Cl | $-OCH_3$ | $-S-CH_2-CO_2C_2H_5$ | " | 3 |
| 38 | H | F | Cl | $-OC_3H_7(i)$ | $-S-CH_2-CO_2CH_3$ | " | 3 |
| 39 | H | F | Cl | $-OCH_3$ | -SH | | 3 |
| 40 | H | F | Cl | $-OC_3H_7(i)$ | -SH | | 3 |
| 41 | H | F | F | $-CO_2C_2H_5$ | $-S-C_6H_5$ | | 3 |
| | | | | | | | ... |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 42 | H | F | F | $-CO_2C_2H_5$ | $-SC_2H_5$ | | 3 |
| 43 | H | Cl | Cl | " | " | | 3 |
| 44 | H | Cl | Cl | " | $-S-(4-Cl-C_6H_4)$ | | 3 |
| 45 | H | Cl | Cl | " | $-S-CH_2CO_2CH_3$ | | 3 |
| 46 | H | Cl | Cl | 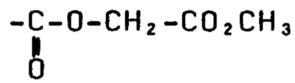 | $-SC_2H_5$ | | 3 |
| 47 | H | Cl | Cl | 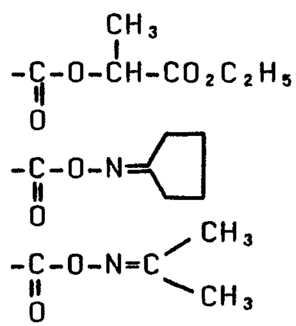 | $-S-(4-Cl-C_6H_4)$ | | 3 |
| 48 | H | Cl | Cl | | $-SC_2H_5$ | | 3 |
| 49 | H | Cl | Cl | | " | | 3 |
| 50 | H | F | F | 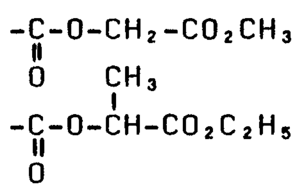 | " | | 3 |
| 51 | H | F | F | | " | | 3 |
| | | | | | | | ... |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 52 | H | F | F | 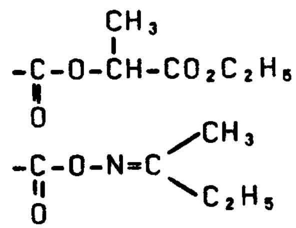 | $-S-(4-Cl-C_6H_4)$ | | 3 |
| 53 | H | F | F | | $-S-C_6H_5$ | | 3 |
| 54 | H | F | F | 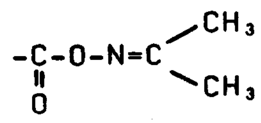 | $-SC_6H_5$ | | 3 |
| 55 | H | H | Cl | $-CO_2C_2H_5$ | $-S-(4-Cl-C_6H_4)$ | | 3 |
| 56 | H | H | Br | $-CO_2C_2H_5$ | $-SC_2H_5$ | | 3 |
| 57 | H | F | Cl | $-O-C_3H_7(i)$ | 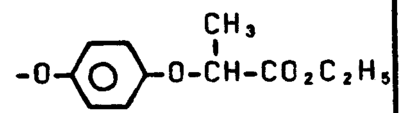 | | 4 |
| 58 | H | F | Cl | $-O-C_3H_7(i)$ | $-O-(4-Cl-C_6H_4)$ | | 4 |
| 59 | H | F | Cl | $-OCH_3$ | $-O-(4-CH_3-C_6H_4)$ | | 4 |
| 60 | H | F | Cl | " | $-O-(4-F-C_6H_4)$ | | 4 |
| 61 | H | F | F | $-CO_2C_2H_5$ | $-O-(4-Cl-C_6H_4)$ | | 4 |
| 62 | H | F | F | $-CO_2C_2H_5$ | $-O-(3-Cl-C_6H_4)$ | | 4 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 63 | H | Cl | Cl | $-CO_2C_2H_5$ | 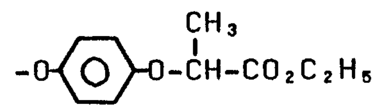 | | 4 |
| 64 | H | F | Cl | $-OCH_3$ | $-OCH_3$ | | 4 |
| 65 | H | F | Cl | $-O-C_3H_7(i)$ | $-OC_2H_5$ | | 4 |
| 66 | H | F | F | $-CO_2C_2H_5$ | $-OCH_3$ | | 4 |
| 67 | H | Cl | Cl | " | " | | 4 |
| 68 | H | F | Cl | $-OCH_3$ | $-O-CH_2-CO_2CH_3$ | | 4 |
| 69 | H | F | Cl | $-O-C_3H_7(i)$ | 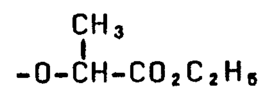 | | 4 |
| 70 | H | H | Cl | $-CO_2C_2H_5$ | $-OCH_3$ | | 4 |
| 71 | H | F | Cl | $-OCH_3$ | $-O-CH_2CH_2-OCH_3$ | | 4 |
| 72 | H | Cl | Cl | 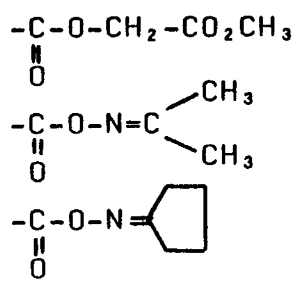 | $-OCH_3$ | | 4 |
| 73 | H | Cl | Cl | | $-O-(4-Cl-C_6H_4)$ | | 4 |
| 74 | H | Cl | Cl | | $-OC_2H_5$ | | 4 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 75 | H | F | F | $-C(=O)-O-CH(CH_3)-CO_2C_2H_5$ | $-O-C_6H_5$ | | 4 |
| 76 | H | F | F | $-C(=O)-O-N=C(CH_3)_2$ | $-OCH_3$ | | 4 |
| 77 | H | F | F | " | $-O-(4-Cl-C_6H_4)$ | | 4 |
| 78 | H | F | F | $-CO_2C_2H_5$ | $-O-C(=O)-CH_3$ | | 5 |
| 79 | H | Cl | Cl | " | " | | 5 |
| 80 | H | Cl | Cl | $-OCH_3$ | " | | 5 |
| 81 | H | F | Cl | $-O-C_3H_7(i)$ | $-O-C(=O)-C_2H_5$ | | 5 |
| 82 | H | F | Cl | $-OCH_3$ | 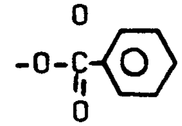 | | 5 |
| 83 | H | F | F | $-CO_2C_2H_5$ | 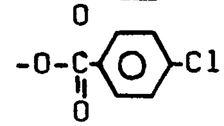 | | 5 |
| 84 | H | F | Cl | $-O-C_3H_7(i)$ | $-O-C(=O)-OCH_3$ | | 6 |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 85 | H | F | F | $-CO_2C_2H_5$ | 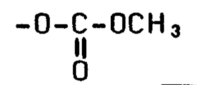 | | 6 |
| 86 | H | F | Cl | $-OCH_3$ | 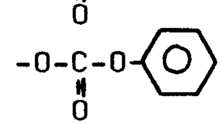 | | 6 |
| 87 | H | F | Cl | " | 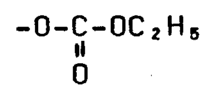 | | 6 |
| 88 | H | Cl | Cl | $-CO_2C_2H_5$ | 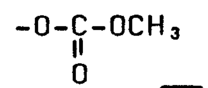 | | 6 |
| 89 | H | F | F | $-CO_2CH_3$ | 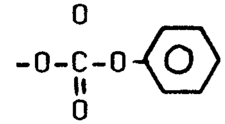 | | 6 |
| 90 | H | H | Cl | $-CO_2C_2H_5$ | 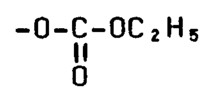 | | 6 |
| 91 | H | F | Cl | $-O-C_3H_7(i)$ | 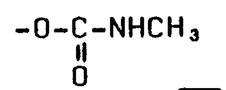 | | 7 |
| 92 | H | F | Cl | " | 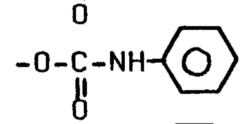 | | 7 |
| 93 | H | F | Cl | $-OCH_3$ | 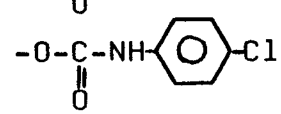 | 224-226 | 7 |
| 94 | H | F | F | $-CO_2C_2H_5$ | " | | 7 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 95 | H | F | F | $-CO_2C_2H_5$ | 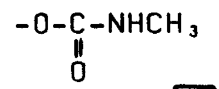 | | 7 |
| 96 | H | Cl | Cl | " | 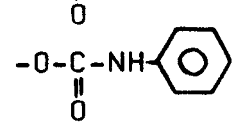 | | 7 |
| 97 | H | Cl | Cl | " | 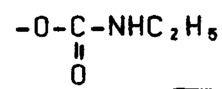 | | 7 |
| 98 | H | H | Cl | " | 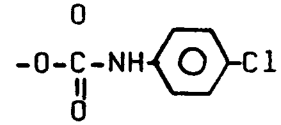 | | 7 |
| 99 | H | F | Cl | $-O-C_3H_7(i)$ | $-N(C_2H_5)_2$ | | 8 |
| 100 | H | F | Cl | $-OCH_3$ | $NH-C_3H_7(i)$ | | 8 |
| 101 | H | F | Cl | " | 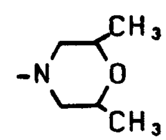 | gelbes Oel | 8 |
| 102 | H | F | Cl | $-O-C_3H_7(i)$ | 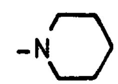 | | 8 |
| 103 | H | F | Cl | $-OCH_3$ | 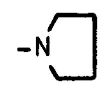 | gelbes Oel | 8 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 104 | H | F | Cl | $-OCH_3$ | 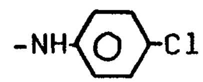 | gelbes Oel | 8 |
| 105 | H | F | Cl | " | 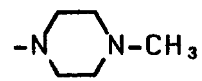 | | 8 |
| 106 | H | F | Cl | " | 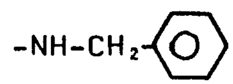 | | 8 |
| 107 | H | F | F | $-CO_2C_2H_5$ | $-N(C_2H_5)_2$ | | 8 |
| 108 | H | F | F | " | 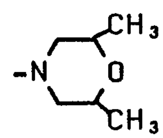 | | 8 |
| 109 | H | F | F | " | 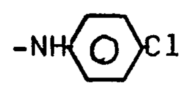 | | 8 |
| 110 | H | Cl | Cl | $-OCH_3$ | $-N(CH_3)_2$ | | 8 |
| 111 | H | Cl | Cl | $-CO_2CH_3$ | $-N(C_2H_5)_2$ | | 8 |
| 112 | H | Cl | Cl | " | 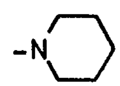 | | 8 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 113 | H | Cl | Cl | $-CO_2C_2H_5$ | -NH- 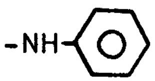 | | 8 |
| 114 | H | F | Cl | $-OC_3H_7(i)$ | $-NHCH_3$ | | 8 |
| 115 | H | F | Cl | $-OCH_3$ | -NH-NH- 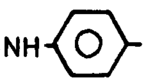 | 135-140 | 8 |
| 116 | H | F | Cl | $-OC_3H_7(i)$ | 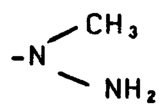 | | 8 |
| 117 | H | Cl | Cl | $-CO_2C_2H_5$ | -NH-NH- 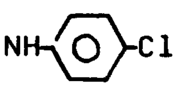 -Cl | | 8 |
| 118 | H | F | Cl | $-OC_3H_7(i)$ | -F | | 9 |
| 119 | H | F | F | $-CO_2C_2H_5$ | -F | | 9 |
| 120 | H | F | F | 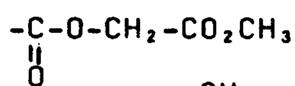 | -F | | 9 |
| 121 | H | F | F | 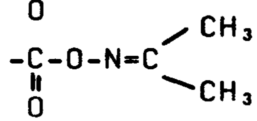 | -F | | 9 |
| 122 | H | Cl | Cl | $-CO_2C_2H_5$ | -F | | 9 |

...

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 123 | H | Cl | Cl | 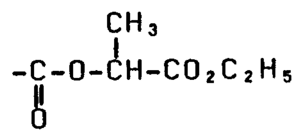 | -F | | 9 |
| 124 | H | Cl | Cl | 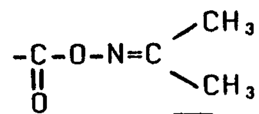 | -F | | 9 |
| 125 | H | Cl | Cl | 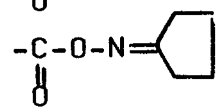 | -F | | 9 |
| 126 | H | H | Cl | $-CO_2C_2H_5$ | -F | | 9 |
| 127 | H | H | Br | 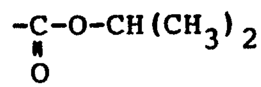 | -OH | 191 - 193 | 1 |
| 128 | H | F | Cl | $-O-CH_2-CO_2CH_3$ | -OH | hellbraunes Öl | 1 |
| 129 | H | F | Cl | $-O-CH_2-CO_2CH_3$ | -Cl | " | 2 |
| 130 | H | F | Cl | 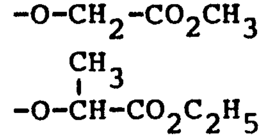 | -OH | " | 1 |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 131 | H | H | F | $-C(=O)-O-CH(CH_3)-CO_2C_2H_5$ | $-OH$ | gelbes Öl | 1 |
| 132 | H | H | F | " | $-Cl$ | hellbraunes Harz | 2 |
| 133 | H | H | F | " | $-SC_2H_5$ | " | 3 |
| 134 | H | H | F | " | $-S-CH_2CO_2C_2H_5$ | " | 3 |
| 136 | H | H | F | " | $-S-CH(CH_3)-CO_2C_2H_5$ | " | 3 |
| 137 | H | H | Cl | " | $-OH$ | " | 1 |
| 138 | H | H | Cl | " | $-Cl$ | " | 2 |
| 139 | H | H | Cl | " | $-SC_2H_5$ | | 3 |
| 140 | H | H | Cl | " | $-S-CH(CH_3)-CO_2C_2H_5$ | | 3 |
| 141 | H | H | Cl | " | $-S-C_6H_5$ | | 3 |
| 142 | H | H | Br | " | $-OH$ | | 1 |
| 143 | H | H | Br | " | $-Cl$ | | 2 |
| 144 | H | H | Br | " | $-SC_2H_5$ | | 3 |
| 145 | H | H | Br | " | $-S-CH_2-CO_2C_2H_5$ | | 3 |
| 146 | H | H | Br | " | $-S-CH(CH_3)-CO_2C_2H_5$ | | 3 |

Fortsetzung Tabelle 1

| Nr. | $R^1$ | $R^2$ | $R^3$ | $R^4$ | A | Fp [°C] | Herstellung analog Bsp. |
|---|---|---|---|---|---|---|---|
| 147 | H | H | Br | $-C(=O)-O-CH(CH_3)-CO_2C_2H_5$ | $-S-C_6H_5$ | | 3 |
| 148 | H | F | Cl | " | $-OH$ | | 1 |
| 149 | H | F | Cl | " | $-Cl$ | | 2 |
| 150 | H | F | Cl | " | $-SC_2H_5$ | | 3 |
| 151 | H | F | Cl | " | $-S-CH(CH_3)-CO_2C_2H_5$ | | 3 |
| 152 | H | F | Br | " | $-OH$ | | 1 |
| 153 | H | F | Br | " | $-Cl$ | | 2 |
| 154 | H | F | Br | " | $-SC_2H_5$ | | 3 |
| 155 | H | F | Br | " | $-S-CH(CH_3)-CO_2C_2H_5$ | | 3 |
| 156 | H | F | Br | " | $-S-C_6H_5$ | | 3 |
| 157 | H | F | Cl | $-OCH_2-C\equiv CH$ | $-OH$ | 136-139° | 1 |
| 158 | H | F | Br | $-OCH_2-C\equiv CH$ | $-OH$ | 138-142 | 1 |

## C. Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60 - 80 %).

Die optische Bonitur der Pflanzen- bzw. Auflaufschäden erfolgte nach dem Auflaufen der Versuchspflanzen nach einer Versuchszeit von 3 - 4 Wochen im Vergleich zu unbehandelten Kontrollen.
Die Boniturwerte in Tabelle 2 zeigen, daß die erfindungsgemäßen Verbindungen eine gute herbizide Vorauflaufwirksamkeit gegen ein breites Spektrum von Ungräsern

...

und Unkräutern besitzen.

Tabelle 2

Vorlaufwirkung der erfindungsgemäßen Verbindungen

| Bsp. | Dosis kg a.i./ha | herbizide Wirkung SIA | CRS | LOM | ECG |
|---|---|---|---|---|---|
| 1 | 2,5 | 5 | 5 | 5 | 5 |
| 64 | 2,5 | 5 | 4 | 5 | 4 |
| 2 | 2,5 | 5 | 5 | 5 | 5 |
| 3 | 2,5 | 5 | 5 | 5 | 5 |
| 10 | 2,5 | 3 | 2 | 4 | 2 |
| 23 | 2,5 | 2 | 5 | 4 | 1 |
| 4 | 2,5 | 5 | 5 | 5 | 5 |
| 35 | 2,5 | 5 | 5 | 5 | 5 |
| 33 | 2,5 | 5 | 5 | 5 | 5 |
| 24 | 2,5 | 5 | 5 | 5 | 5 |
| 11 | 2,5 | 5 | 5 | 5 | 5 |
| 127 | 2,5 | 5 | 5 | 5 | 5 |
| 13 | 2,5 | 5 | 5 | 5 | 3 |
| 128 | 2,5 | 5 | 5 | 5 | 5 |
| 17 | 2,5 | 4 | 2 | 5 | 5 |
| 7 | 2,5 | 5 | 5 | 4 | 3 |
| 8 | 2,5 | 4 | 5 | 5 | 5 |
| 9 | 2,5 | 5 | 5 | 5 | 5 |
| 18 | 2,5 | 5 | 2 | 5 | 2 |
| 26 | 2,5 | 3 | 5 | 2 | 3 |
| 27 | 2,5 | 5 | 4 | 5 | 4 |
| 37 | 2,5 | 5 | 5 | 5 | 5 |
| 101 | 2,5 | 4 | 5 | 5 | 5 |
| 130 | 2,5 | 5 | 5 | 3 | 5 |
| 157 | 2,5 | 5 | 5 | 5 | 5 |

Abkürzungen:

SIA = Sinapis arvensis
CRS = Chrysanthemum segetum
LOM = Lolium multiflorum
ECG = Echinochloa crus-galli
a.i. = Aktivsubstanz

## 2. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.
Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1 °C, relative Luftfeuchte 60 - 80 %) die Wirkung der Präparate optisch im Vergleich zu unbehandelten Kontrollen bonitiert.
Die erfindungsgemäßen Verbindungen weisen auch im Nachauflauf eine gute herbizide Wirksamkeit gegen ein breites Spektrum wirtschaftlich wichtiger Ungräser und Unkräuter auf.

Tabelle 3

Nachauflaufverfahren der erfindungsgemäßen Verbindungen

| Bsp. | Dosis kg a.i./ha | herbizide Wirkung SIA | CRS | LOM | ECG |
|---|---|---|---|---|---|
| 1 | 2,5 | 5 | 4 | 5 | 5 |
| 64 | 2,5 | 5 | 3 | 3 | 2 |
| 2 | 2,5 | 5 | 3 | 5 | 5 |
| 3 | 2,5 | 5 | 4 | 4 | 4 |
| 4 | 2,5 | 5 | 3 | 1 | 2 |
| 35 | 2,5 | 5 | 5 | 4 | 3 |

...

Fortsetzung Tabelle 3

| Bsp. | Dosis kg a.i./ha | herbizide Wirkung SIA | CRS | LOM | ECG |
|---|---|---|---|---|---|
| 33 | 2,5 | 5 | 5 | 3 | 2 |
| 24 | 2,5 | 5 | 4 | 4 | 3 |
| 11 | 2,5 | 5 | 2 | 4 | 2 |
| 127 | 2,5 | 4 | 2 | 4 | 5 |
| 13 | 2,5 | 4 | 3 | 3 | 2 |
| 128 | 2,5 | 3 | 3 | 3 | 4 |
| 7 | 2,5 | 5 | 5 | 2 | 5 |
| 8 | 2,5 | 4 | 3 | 2 | 4 |
| 9 | 2,5 | 4 | 4 | 4 | 3 |
| 18 | 2,5 | 4 | 1 | 3 | 3 |
| 37 | 2,5 | 4 | 5 | 4 | 4 |
| 130 | 2,5 | 4 | 4 | 2 | 2 |
| 157 | 2,5 | 5 | 5 | 4 | 4 |

Abkürzungen: siehe Tabelle 2

3. Kulturpflanzenverträglichkeit

In weiteren Versuchen im Gewächshaus wurden Samen einer größeren Anzahl von Kulturpflanzen in Töpfen in sandigem Lehmboden ausgelegt und mit Erde abgedeckt.

Ein Teil der Töpfe wurde sofort wie unter 1. beschrieben behandelt; die übrigen wurden im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z. B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

0215424

Patentansprüche:

HOE 85/F 201

1. Verbindungen der Formel I

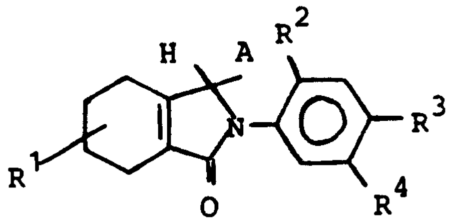

(I)

worin

$R^1$ = $(C_1-C_4)$-Alkyl oder Wasserstoff

$R^2$ = Wasserstoff oder Halogen

$R^3$ = Halogen

$R^4$ = Hydroxy, $(C_1-C_4)$-Alkoxy, $(C_1-C_4)$Alkoxy-$(C_1-C_4)$-alkoxy, $(C_3-C_6)$-Cycloalkoxy, $(C_3-C_6)$-Alkenyloxy, $(C_3-C_6)$-Alkinyloxy, $(C_1-C_4)$-Alkylthio, $(C_1-C_4)$-Alkylsulfinyl, $(C_1-C_4)$-Alkylsulfonyl, $NO_2$, CN, Halogen-$(C_1-C_4)$-alkyl mit einem oder mehreren Halogenatomen, Mono-Cyano-$(C_1-C_4)$-alkyl, Phenoxy, Phenoxy-$(C_1-C_4)$-alkyl, Phenyl-$(C_1-C_4)$-alkoxy Phenylthio, Phenylsulfinyl, Phenylsulfonyl, wobei in den 6 letztgenannten Resten der Phenylring bis zu dreifach durch Halogen, $(C_1-C_4)$-Alkyl, $(C_1-C_4)$-Alkoxy oder $(C_1-C_4)$-Alkoxycarbonyl substituiert sein kann,

eine Gruppe der Formeln

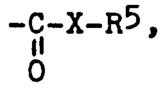, 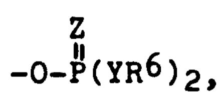, 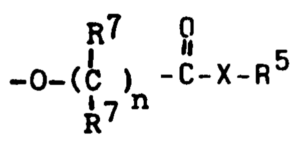 oder

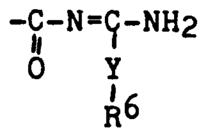 ,

X = O, S oder $NR^8$,

Y,Z = unabhängig voneinander O oder S

$R^5$ = Wasserstoff, $(C_1-C_{12})$-Alkyl, das bis zu 6-fach durch Halogen und/oder bis zu dreifach durch $(C_1-C_4)$-Alkoxy, $(C_1-C_4)$-Alkoxy-$(C_1-C_4)$-alkoxy,

$(C_1-C_4)$-Alkoxycarbonyl, $(C_1-C_4)$-Alkylthio, $(C_1-C_4)$-Alkylsulfonyl, $(C_1-C_4)$-Alkylsulfinyl, $(C_1-C_4)$-Alkylamino, Di- $(C_1-C_4)$-alkylamino, Furyl, Tetrahydrofuryl, Benzofuryl, Phenyl, Phenoxy, Benzyloxy, wobei die drei letztgenannten Reste im Phenylteil bis zu zweifach durch Halogen, $(C_1-C_4)$-Alkyl oder $(C_1-C_4)$-Alkoxy substituiert sein können, substituiert sein kann, $(C_3-C_6)$-Cycloalkyl, $(C_3-C_6)$-Alkenyl, Cyclohexenyl, $(C_3-C_6)$-Alkinyl, Phenyl, das bis zu dreifach durch Halogen, $(C_1-C_4)$-Alkyl, $(C_1-C_4)$-Alkoxy oder $(C_1-C_4)$-Alkoxycarbonyl substituiert sein kann,

ein in der Landwirtschaft einsetzbares Kation oder einen Rest der Formel $-N=CR^9R^{10}$, wobei für den letztgenannten Rest $R^4$ = $-CO-OR^5$ bedeuten muß,

$R^6$ = unabhängig voneinander $(C_1-C_4)$-Alkyl oder Benzyl

$R^7$ = unabhängig voneinander Wasserstoff, $(C_1-C_4)$-Alkyl oder $-CCl_3$,

$R^8$ = Wasserstoff, $(C_1-C_4)$-Alkyl, das zusammen mit $R^5$ und dem diese Reste verbindenen Stickstoffatom einen fünf- bis siebengliedrigen Heterocyclus bilden kann, der als Ringglieder 1 oder 2 Reste der Gruppe -O-, -S- oder $-NR^6-$ enthält und der bis zu dreifach durch $(C_1-C_4)$-Alkyl substituiert sein kann,

$R^9, R^{10}$ = unabhängig voneinander $(C_1-C_4)$-Alkyl, $(C_3-C_6)$-Cycloalkyl $(C_1-C_4)$Alkoxycarbonyl, oder gemeinsam eine $(C_2-C_7)$-Alkylenkette,

A = OH, SH, Halogen, CN, einen Rest der Formeln $-OR^{11}$, $-S(O)_pR^{11}$, $-NR^{12}R^{13}$ oder $-NH-NR^{14}R^{15}$,

$R^{11}$ = $(C_1-C_{12})$-Alkyl, $(C_1-C_4)$-Alkoxy-$(C_1-C_4)$-alkyl, Phenoxy$(C_1-C_4)$alkyl, Benzyloxy$(C_1-C_4)$alkyl, wobei die beiden letztgenannten Reste im Phenylring bis zu dreifach durch Halogen, $(C_1-C_4)$-Alkyl oder $(C_1-C_4)$-Alkoxy substituiert sein können,

($C_1$-$C_4$)-Alkoxycarbonyl-($C_1$-$C_4$)-alkyl, Phenyl oder Benzyl, die beide bis zu dreifach im Phenylring durch Halogen, ($C_1$-$C_4$)-Alkyl, ($C_1$-$C_4$)-Alkoxy, ($C_1$-$C_4$)-Alkoxycarbonyl, ($C_1$-$C_4$)-Alkoxycarbonyl-($C_1$-$C_4$)-alkoxy, $CF_3$ oder $NO_2$ substituiert sein können,
eine Gruppe der Formeln
$-\underset{\substack{\| \\ O}}{C}-R^{16}$, $-\underset{\substack{\| \\ O}}{C}-OR^{16}$ oder $-\underset{\substack{\| \\ O}}{C}-NH-R^{16}$,

$R^{12}$,$R^{13}$ = unabhängig voneinander Wasserstoff, ($C_1$-$C_4$)-Alkyl, oder zusammen mit dem sie verbindenden Stickstoffatom einen 3- bis 7-gliedrigen Ring bilden, wobei eine Methylengruppe durch O, S oder $NR^7$ ersetzt sein kann und der bis zu dreifach durch ($C_1$-$C_4$)-Alkyl substituiert sein kann,
Phenyl oder Benzyl, die beide im Phenylring bis zu dreifach durch Halogen, ($C_1$-$C_4$)-Alkyl, ($C_1$-$C_4$)-Alkoxy, ($C_1$-$C_4$)-Alkoxycarbonyl, $CF_3$ oder $NO_2$ substituiert sein können,

$R^{14}$,$R^{15}$ = unabhängig voneinander Wasserstoff, ($C_1$-$C_4$)-Alkyl oder Phenyl, das bis zu dreifach durch Halogen oder ($C_1$-$C_4$)-Alkyl substituiert sein kann,

$R^{16}$ = ($C_1$-$C_4$)-Alkyl, das bis zu dreifach durch Halogen, ($C_1$-$C_4$)-Alkoxy, ($C_1$-$C_4$)-Alkylthio, CN, Phenyl oder Phenyl, das bis zu zweifach durch Halogen, ($C_1$-$C_4$)-Alkyl, ($C_1$-$C_4$)-Alkoxy, $CF_3$, $NO_2$ substituiert ist, substituiert sein kann, oder
Phenyl, das ein- bis dreifach durch Halogen, ($C_1$-$C_4$)-Alkyl, ($C_1$-$C_4$)-Alkoxy, ($C_1$-$C_4$)-Alkoxycarbonyl, $CF_3$ oder $NO_2$ substituiert sein kann,

n = die Zahl 1, 2 oder 3 und

p = die Zahl 0, 1 oder 2

bedeuten.

2. Verfahren zur Herstellung von Verbindungen der Formel I, dadurch gekennzeichnet, daß man
a) eine Verbindung der Formel II

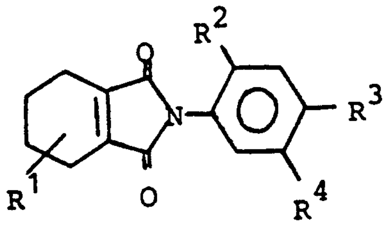

(II)

reduziert und

b) die unter a) erhaltenen Verbindungen der Formel I mit A = OH gegebenenfalls durch Umsetzung $b_1$) mit einer Verbindung der Formel $Cl-CO-R^{16}$, $Cl-CO-OR^{16}$ oder $R^{16}-NCO$ in Gegenwart einer Base oder $b_2$) mit einer Verbindung der Formel $R^{11}-Hal$ (Hal = Cl, Br, J) in Gegenwart einer Base, oder $b_3$) mit einem Isocyanat $R^{16}-N=C=O$ gegebenenfalls in Gegenwart einer Base, oder $b_4$) mit einem anorganischen Säurechlorid wie $SOCl_2$, $POCl_3$, $PCl_3$ in andere Verbindungen der Formel I überführt und die unter $b_4$) erhaltenen Verbindungen der Formel I gegebenenfalls mit einem Alkalimetallfluorid, -bromid oder -jodid oder mit einem Alkalicyanid oder mit einer Verbindung der Formel $HOR^{11}$, $HSR^{11}$, $HNR^{12}R^{13}$, oder $H_2N-NR^{14}R^{15}$ umsetzt oder die unter $b_1$) erhaltenen Verbindungen im Falle $A = S-R^{11}$ gegebenenfalls oxidiert.

3. Verwendung der Verbindungen der Formel I als Herbizide.

4. Herbizide Mittel, dadurch gekennzeichnet, daß sie eine Verbindung der Formel I von Anspruch 1 enthalten.

5. Verfahren zur Bekämpfung von unerwünschten Pflanzen in Nutzpflanzenkulturen, dadurch gekennzeichnet, daß man eine wirksame Menge einer Verbindung der Formel I von Anspruch 1 auf die Schadpflanzen oder die Anbaufläche aufbringt.

**PATENTANSPRÜCHE** (Vertragsstaat AT)

1. Verfahren zur Herstellung von Verbindungen der Formel I,

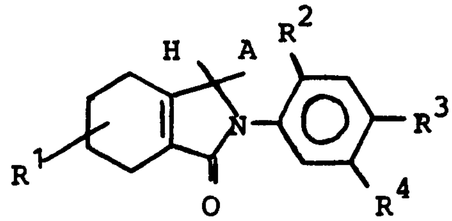

(I),

worin

$R^1$ = $(C_1-C_4)$-Alkyl oder Wasserstoff

$R^2$ = Wasserstoff oder Halogen

$R^3$ = Halogen

$R^4$ = Hydroxy, $(C_1-C_4)$-Alkoxy, $(C_1-C_4)$Alkoxy-$(C_1-C_4)$-alkoxy, $(C_3-C_6)$-Cycloalkoxy, $(C_3-C_6)$-Alkenyloxy, $(C_3-C_6)$-Alkinyloxy, $(C_1-C_4)$-Alkylthio, $(C_1-C_4)$-Alkylsulfinyl, $(C_1-C_4)$-Alkylsulfonyl, $NO_2$, CN, Halogen-$(C_1-C_4)$-alkyl mit einem oder mehreren Halogenatomen, Mono-Cyano-$(C_1-C_4)$-alkyl, Phenoxy, Phenoxy-$(C_1-C_4)$alkyl, Phenyl-$(C_1-C_4)$-alkoxy Phenylthio, Phenylsulfinyl, Phenylsulfonyl, wobei in den 6 letztgenannten Resten der Phenylring bis zu dreifach durch Halogen, $(C_1-C_4)$-Alkyl, $(C_1-C_4)$-Alkoxy oder $(C_1-C_4)$-Alkoxycarbonyl substituiert sein kann,

eine Gruppe der Formeln

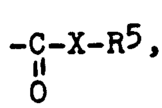, 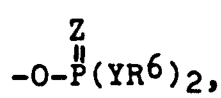, 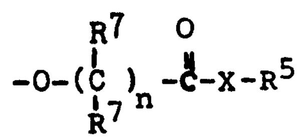 oder

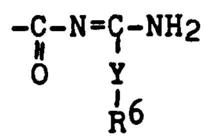 ,

X = O, S oder $NR^8$,

Y,Z = unabhängig voneinander O oder S

$R^5$ = Wasserstoff, $(C_1-C_{12})$-Alkyl, das bis zu 6-fach durch Halogen und/oder bis zu dreifach durch $(C_1-C_4)$-Alkoxy, $(C_1-C_4)$-Alkoxy-$(C_1-C_4)$-alkoxy,

$(C_1-C_4)$-Alkoxycarbonyl, $(C_1-C_4)$-Alkylthio, $(C_1-C_4)$-Alkylsulfonyl, $(C_1-C_4)$-Alkylsulfinyl, $(C_1-C_4)$-Alkylamino, Di- $(C_1-C_4)$-alkylamino, Furyl, Tetrahydrofuryl, Benzofuryl, Phenyl, Phenoxy, Benzyloxy, wobei die drei letztgenannten Reste im Phenylteil bis zu zweifach durch Halogen, $(C_1-C_4)$-Alkyl oder $(C_1-C_4)$-Alkoxy substituiert sein können, substituiert sein kann,
$(C_3-C_6)$-Cycloalkyl, $(C_3-C_6)$-Alkenyl, Cyclohexenyl, $(C_3-C_6)$-Alkinyl, Phenyl, das bis zu dreifach durch Halogen, $(C_1-C_4)$-Alkyl, $(C_1-C_4)$-Alkoxy oder $(C_1-C_4)$-Alkoxycarbonyl substituiert sein kann,

ein in der Landwirtschaft einsetzbares Kation oder einen Rest der Formel $-N=CR^9R^{10}$, wobei für den letztgenannten Rest $R^4 = -CO-OR^5$ bedeuten muß,

$R^6$ = unabhängig voneinander $(C_1-C_4)$-Alkyl oder Benzyl

$R^7$ = unabhängig voneinander Wasserstoff, $(C_1-C_4)$-Alkyl oder $-CCl_3$,

$R^8$ = Wasserstoff, $(C_1-C_4)$-Alkyl, das zusammen mit $R^5$ und dem diese Reste verbindenen Stickstoffatom einen fünf- bis siebengliedrigen Heterocyclus bilden kann, der als Ringglieder 1 oder 2 Reste der Gruppe -O-, -S- oder $-NR^6-$ enthält und der bis zu dreifach durch $(C_1-C_4)$-Alkyl substituiert sein kann,

$R^9,R^{10}$ = unabhängig voneinander $(C_1-C_4)$-Alkyl, $(C_3-C_6)$-Cycloalkyl $(C_1-C_4)$Alkoxycarbonyl, oder gemeinsam eine $(C_2-C_7)$-Alkylenkette,

A = OH, SH, Halogen, CN,
einen Rest der Formeln
$-OR^{11}$, $-S(O)_pR^{11}$, $-NR^{12}R^{13}$ oder $-NH-NR^{14}R^{15}$,

$R^{11}$ = $(C_1-C_{12})$-Alkyl, $(C_1-C_4)$-Alkoxy-$(C_1-C_4)$-alkyl, Phenoxy$(C_1-C_4)$alkyl, Benzyloxy$(C_1-C_4)$alkyl, wobei die beiden letztgenannten Reste im Phenylring bis zu dreifach durch Halogen, $(C_1-C_4)$-Alkyl oder $(C_1-C_4)$-Alkoxy substituiert sein können,

$(C_1-C_4)$-Alkoxycarbonyl-$(C_1-C_4)$-alkyl, Phenyl oder Benzyl, die beide bis zu dreifach im Phenylring durch Halogen, $(C_1-C_4)$-Alkyl, $(C_1-C_4)$-Alkoxy, $(C_1-C_4)$-Alkoxycarbonyl, $(C_1-C_4)$-Alkoxycarbonyl-$(C_1-C_4)$-alkoxy, $CF_3$ oder $NO_2$ substituiert sein können,
eine Gruppe der Formeln
$-\underset{\underset{O}{\|}}{C}-R^{16}$, $-\underset{\underset{O}{\|}}{C}-OR^{16}$ oder $-\underset{\underset{O}{\|}}{C}-NH-R^{16}$,

$R^{12}$,$R^{13}$=unabhängig voneinander Wasserstoff, $(C_1-C_4)$-Alkyl, oder zusammen mit dem sie verbindenden Stickstoffatom einen 3- bis 7-gliedrigen Ring bilden, wobei eine Methylengruppe durch O, S oder $NR^7$ ersetzt sein kann und der bis zu dreifach durch $(C_1-C_4)$-Alkyl substituiert sein kann,
Phenyl oder Benzyl, die beide im Phenylring bis zu dreifach durch Halogen, $(C_1-C_4)$-Alkyl, $(C_1-C_4)$-Alkoxy, $(C_1-C_4)$-Alkoxycarbonyl, $CF_3$ oder $NO_2$ substituiert sein können,

$R^{14}$,$R^{15}$=unabhängig voneinander Wasserstoff, $(C_1-C_4)$-Alkyl oder Phenyl, das bis zu dreifach durch Halogen oder $(C_1-C_4)$-Alkyl substituiert sein kann,

$R^{16}$ = $(C_1-C_4)$-Alkyl, das bis zu dreifach durch Halogen, $(C_1-C_4)$-Alkoxy, $(C_1-C_4)$-Alkylthio, CN, Phenyl oder Phenyl, das bis zu zweifach durch Halogen, $(C_1-C_4)$-Alkyl, $(C_1-C_4)$-Alkoxy, $CF_3$, $NO_2$ substituiert ist, substituiert sein kann, oder
Phenyl, das ein- bis dreifach durch Halogen, $(C_1-C_4)$-Alkyl, $(C_1-C_4)$-Alkoxy, $(C_1-C_4)$-Alkoxycarbonyl, $CF_3$ oder $NO_2$ substituiert sein kann,

n = die Zahl 1, 2 oder 3 und

p = die Zahl 0, 1 oder 2

bedeuten,

dadurch gekennzeichnet, daß man

a) eine Verbindung der Formel II

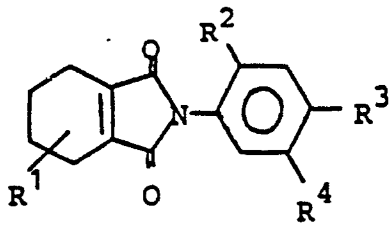

(II)

reduziert und

b) die unter a) erhaltenen Verbindungen der Formel I mit A = OH gegebenenfalls durch Umsetzung $b_1$) mit einer Verbindung der Formel $Cl-CO-R^{16}$, $Cl-CO-OR^{16}$ oder $R^{16}-NCO$ in Gegenwart einer Base oder $b_2$) mit einer Verbindung der Formel $R^{11}-Hal$ (Hal = Cl, Br, J) in Gegenwart einer Base, oder $b_3$) mit einem Isocyanat $R^{16}-N=C=O$ gegebenenfalls in Gegenwart einer Base, oder $b_4$) mit einem anorganischen Säurechlorid wie $SOCl_2$, $POCl_3$, $PCl_3$ in andere Verbindungen der Formel I überführt und die unter $b_4$) erhaltenen Verbindungen der Formel I gegebenenfalls mit einem Alkalimetallfluorid, -bromid oder -jodid oder mit einem Alkalicyanid oder mit einer Verbindung der Formel $HOR^{11}$, $HSR^{11}$, $HNR^{12}R^{13}$, oder $H_2N-NR^{14}R^{15}$ umsetzt oder die unter $b_1$) erhaltenen Verbindungen im Falle A = $S-R^{11}$ gegebenenfalls oxidiert.

2. Verwendung der Verbindungen der Formel I als Herbizide.

3. Herbizide Mittel, dadurch gekennzeichnet, daß sie eine Verbindung der Formel I von Anspruch 1 enthalten.

4. Verfahren zur Bekämpfung von unerwünschten Pflanzen in Nutzpflanzenkulturen, dadurch gekennzeichnet, daß man eine wirksame Menge einer Verbindung der Formel I von Anspruch 1 auf die Schadpflanzen oder die Anbaufläche aufbringt.